# EUROPEAN PATENT APPLICATION

(11) **EP 4 502 603 A1**
(43) Date of publication of application: **05.02.2025**
(21) Application number: 23773467.8
(22) Date of filing: 07.02.2023
(51) Int. Cl.: G01N 33/531, G01N 33/558, G01N 33/569

(54) **BIOLOGICAL SAMPLE EXTRACT SOLUTION, AND APPLICATION AND USE METHOD THEREOF**

(30) Priority: 25.03.2022 CN 202210305651
(71) Applicant: Leadway (HK) Limited, Sheung Wan, Hong Kong (CN)
(72) Inventor: SHANG, Tao, Hangzhou, Zhejiang 310030 (CN); FEI, Fengqin, Hangzhou, Zhejiang 310030 (CN); KONG, Jianxi, Hangzhou, Zhejiang 310030 (CN); ZHENG, Zhenzhen, Hangzhou, Zhejiang 310030 (CN); TONG, Fangli, Hangzhou, Zhejiang 310030 (CN); WU, Silu, Hangzhou, Zhejiang 310030 (CN); CUI, Yaru, Hangzhou, Zhejiang 310030 (CN)
(74) Representative: Gulde & Partner
(86) International application number: PCT/CN2023/074724
(87) International publication number: WO 2023/179216

(57) **Abstract**

The present invention provides a biological sample extraction buffer, and an application and a method of use thereof. The biological sample extraction buffer includes a surfactant and a salt ion. The surfactant is selected from Triton X-100 or Tween-20. The biological sample extraction buffer not only can improve the test sensitivity of immunochromatographic antigen test products, but also can improve the inactivation capability of the extraction buffer against various viruses such as SARS-CoV-2. It plays an important role in preventing secondary contamination and protecting population.

## Description

### Field of the Invention

The present invention belongs to the technical field of biological testing, in particular to a virus sample extraction buffer, and a method of use and an application thereof.

### Background of the Invention

A virus (biological virus) is a non-cellular organism with tiny size and simple structure, which must be parasitized in a living cell and proliferate in a replicating manner. It consists of a protein shell and an internal genetic material, nucleic acid (DNA or RNA). Viruses cannot survive on their own and must live inside the cells of other organisms.

Currently, nucleic acid test methods or antigen/antibody test methods are usually used for virus test. The virus in the sample is broken down using an extraction buffer, then the nucleic acid material broken down is analyzed using the nucleic acid test method, and the proteins obtained from breaking down are analyzed using the antigen test method, so as to determine whether the corresponding virus is present in the sample or not.

The test of the currently popular SARS-CoV-2 is taken as an example. A swab is used to collect a sample from specific parts such as the mouth and nose, and then the swab with the collected sample is submerged in a virus extraction buffer for subsequent test and analysis.

After the end of the test, there is usually a residual amount of the extraction buffer that holds the virus sample. In the process of treating the remaining extraction buffer, whether the collected sample is completely inactivated or not is a risk encountered in the test process. Virus samples that are not completely inactivated pose a risk of secondary infection to staff during transfer and treatment. Particularly in terms of the treatment on highly infectious and pathogenic viruses such as SARS-CoV-2, it is necessary to effectively inactivate the viruses in the samples in order to block the transmission routes of epidemic diseases resulted from secondary infection.

Direct addition of an inactivating agent such as a guanidine salt to the extraction buffer is one of the current methods for inactivation of a virus sample. However, the guanidine salt will cause protein denaturation, and thus is not applicable to antigen test methods taking proteins as test objects.

The SARS-CoV-2 antigen test at present has been popularized and used in community population due to its characteristics of simple test operation and rapid obtaining of results, and the purpose of rapid population screening is realized through self-test of community population. How to quickly and effectively inactivate these remaining samples collected and tested by non-professionals to avoid the exposure of these infectious and pathogenic virus samples is something that needs to be studied in depth by the developers of viral antigen test reagents, and it is of important significance to develop extraction buffers that can effectively inactivate virus samples.

### Summary of the Invention

In order to solve the above problems in the prior art, the present invention provides a biological sample (virus) extraction buffer, comprising a surfactant and a salt ion, the surfactant being selected from Triton X-100 or Tween-20.

Further, the concentration of the surfactant is 0.5% to 2%(w/V).

Further, the concentration of the salt ion is 0.9% to 5%(w/V).

Further, the salt ion is selected from NaCl or KCl.

Further, the pH of the extraction buffer is 8.0-9.0.

The present invention further provides an application of the virus extraction buffer in extraction of a virus protein sample.

Further, the biological sample is a virus, and still further, the virus is selected from SARS-CoV-2.

The present invention further provides a method of use of the biological sample extraction buffer, comprising the following steps: directly adding a swab sample or a liquid sample into a certain volume of a virus extraction buffer for incubation and extraction, and dropwise adding the extracted sample to a test device to interpret the result.

Further, the swab sample or the liquid sample is incubated in the virus extraction buffer for 30s to 1min.

Further, the test device comprises an upper cover and a lower plate, and a test strip is assembled between the upper cover and the lower plate.

The biological sample extraction buffer described in the present invention not only can improve the test sensitivity of immunochromatographic antigen test products, but also improves the inactivation capability of the extraction buffer against viruses (for example, various viruses such as SARS-CoV-2). This is conducive to the rapid and effective inactivation of samples collected by non-professionals on their own, avoiding the exposure of infectious and pathogenic virus samples, and playing an important role in preventing secondary contamination and protecting population.

### Brief Description of the Drawings

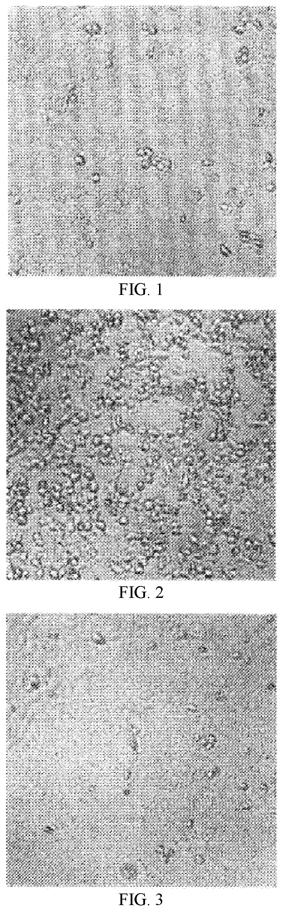

FIG. 1 is a photo under microscope examination of the negative control of Example 2.
FIG. 2 is a photo under microscope examination of the positive control of Example 2.
FIG. 3 is a photo under microscope examination of the experimental group of Example 2.
FIG. 4 shows the extraction buffer contained in an extracting tube.
FIG. 5 shows a reagent plate installed in the extracting tube.
FIG. 6 shows swab sampling and test steps.

### Detailed Description of the Embodiments

The present invention will be further illustrated below in conjunction with the specific embodiments. However, the embodiments below are merely for illustration of the present invention, instead of limiting the scope of the present invention.

The present invention provides an extraction buffer having an inactivation effect, which comprises a surfactant and a salt ion. The surfactant is selected from, but not limited to, Triton X-100 or Tween-20, and the salt ion is selected from, but not limited to, sodium chloride or potassium chloride. In a preferred example, the salt ion in the extraction buffer is a salt ion at high concentration.

### Example 1

Experimental materials:
1. Formulation of an extraction buffer:
   A surfactant selected from Triton X-100 or Tween-20 or SDS.
   A salt ion selected from NaCl or KCl.
2. COVID-19 antigen test kit (COVID-19 Antigen Rapid Test Kit (Latex) provided by ACON)
3. Recombinant antigen of SARS-CoV-2 N protein
4. SARS-CoV-2 strain

Experimental method:
1. Different concentrations of extraction buffers were prepared from a surfactant Triton X-100, Tween-20 or SDS and a salt ion NaCl or KCl at different ratios with purified water. The pH of the extraction buffer is 8.0-9.0. Specific formulations were as shown in Table 1.

**Table 1 Table of formulations of extraction buffers**

| Number of Formulation | Surfactant (w/V) | Salt (w/V) |
|---|---|---|
| **1** | 0.1% Triton X-100 | 0.5% NaCl |
| **2** | 0.1% Tween 20 | 0.5% KCl |
| **3** | 0.1% SDS | 0.5% NaCl |
| **4** | 0.5% Triton X-100 | 0.9% NaCl |
| **5** | 0.5% Tween 20 | 0.9% KCl |
| **6** | 0.5% SDS | 0.9% NaCl |
| **7** | 1% Triton X-100 | 3% NaCl |
| **8** | 1% Tween 20 | 3% KCl |
| **9** | 1% SDS | 3% NaCl |
| **10** | 2% Triton X-100 | 5% NaCl |
| **11** | 2% Tween 20 | 5% KCl |
| **12** | 2% SDS | 5% NaCl |

| | | |
|---|---|---|
| * (m/V) represents grams of a solute dissolved in per 100ml of a solvent | | |

2. The extraction buffers with formulations in Table 1 were used to prepare the same concentration (low concentration and medium concentration) of quality control products of the recombinant antigen of the SARS-CoV-2 N protein, respectively, and their sensitivity tests were carried out using a COVID-19 Antigen Rapid Test Kit. As for the low concentration: the recombinant antigen of the SARS-CoV-2 N protein has a concentration of 20pg/mL; and as for the medium concentration: the recombinant antigen of the SARS-CoV-2 N protein has a concentration of 60pg/mL. The results of the experiment were shown in Table 2.

**Table 2 Experimental results of sensitivity**

| Assessment of sensitivity | Sample at low concentration | Sample at medium concentration |
|---|---|---|
| Formulation 1 | L3 | L5 |
| Formulation 2 | L3 | L5 |
| Formulation 3 | L1 | L2 |
| Formulation 4 | L4 | L6 |
| Formulation 5 | L4 | L6 |
| Formulation 6 | L1 | L2 |
| Formulation 7 | L5 | L7 |
| Formulation 8 | L5 | L7 |
| Formulation 9 | L2 | L3 |
| Formulation 10 | L5 | L7 |
| Formulation 11 | L5 | L7 |
| Formulation 12 | L2 | L3 |

| | | |
|---|---|---|
| *L represents the intensity of color rendering, and from L1 to L10, a higher subsequent numeric value indicates stronger color rendering of the product test line. The value of L at low concentration needs to reach L3, while it will be better if the value of L at medium concentration is higher. | | |

3. A formulation with high sensitivity in step 2 was selected to prepare an extraction buffer, which diluted the concentration of the SARS-CoV-2 strain to 1.0*10⁶ TCID₅₀/mL.
4. After incubation for 60 minutes at room temperature, the virus treated with the extraction buffer was diluted with a culture medium at a ratio of 1:10,000 to serve as an experimental group. The extraction buffer with no virus was diluted with a culture medium at a ratio of 1:10,000 to serve as a negative control group, and the virus at a concentration of 1.0*10⁶ TCID₅₀/mL was directly diluted with a culture medium at a ratio of 1:10,000 to serve as a positive control group.
5. The Vero E6 cell strain in culture wells of a culture plate was cultured to a density of about 80%, the virus solutions of the experimental group, the negative control group and the positive control group in step 4 were transferred to the culture wells, and the culture plate was placed in a 5% CO₂ incubator at 37°C for 3 days.
6. The production status of the cells was observed and the inactivation effect of the extraction buffer was assessed.

### Example 2 Inactivation experiments of the extraction buffer

Formulation 7 of Example 1 was selected as the extraction buffer, and the inactivation experiment of the virus was carried out according to the method in Example 1. The results of the experiment were shown in Table 3 below.

**Table 3 Results of virus inactivation experiments**

| | Photo under microscope | Result |
|---|---|---|
| Negative control group | See FIG. 1 | N/D |
| Positive control group | See FIG. 2 | CPE |
| Inactivated result (experimental group) | See FIG. 3 | Non CPE |

Result interpretation: N/D indicates no virus is found; CPE indicates survival of viruses; and Non-CPE indicates inactivation of viruses.

Extraction buffers prepared from other formulations with high sensitivity were selected for inactivation experiments, achieving the same inactivation effect.

### Example 3

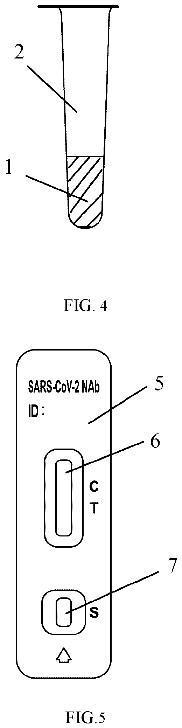

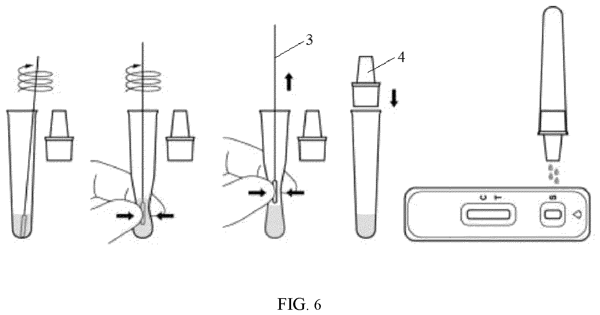

The experimental procedures were as follows: as shown in FIG. 4, FIG. 5 and FIG. 6, an extracting tube 2 containing the extraction buffer 1 was opened, the extraction buffer in the extracting tube using Formulation 7 in Table 1; the swab 3 after sampling was inserted into the extracting tube containing the extraction buffer, the swab was rotated inside the extracting tube for 30 seconds, and then the head of the swab was squeezed against the wall of the tube for 5 times to ensure that the sample on the swab was sufficiently eluted; the head of the swab was squeezed along the inner wall of the extracting tube so that the extraction buffer was retained in the tube as much as possible, and the swab was taken out and discarded to obtain a treated sample extract; a trickle emitter 4 was capped into the extracting tube; 4-5 drops of the treated sample extract were dropped to a sample adding hole7 in a test device 5; and the test results were interpreted and displayed in the observation window 6 of the test device. The test device comprised an upper cover and a lower plate, and a test strip was assembled between the upper cover and the lower plate.

Swabs were used to collect samples of positive patients and negative persons confirmed by the nucleic acid test, and sampling, extraction of the extraction buffer and test of a sample added to the test device were carried out according to the steps described in this Example 4. The test results were consistent with the nucleic acid test results.

A surfactant such as Triton X-100 or Tween 20 at 0.5% to 2% (w/V) and a salt ion (such as NaCl, KC1) at a high concentration of 0.9% to 5% (w/V) were added to the extraction buffer described in the present invention, thus not only improving the test sensitivity of immunochromatographic products, but also realizing the inactivation of the virus for a certain period of time (e.g., within 60 minutes). It played an important role in preventing secondary contamination of the remaining sample and protecting population.

## Claims

1. A biological sample extraction buffer, comprising a surfactant and a salt ion, the surfactant being selected from Triton X-100 or Tween-20.

2. The biological sample extraction buffer according to claim 1, wherein the concentration of the surfactant is 0.5% to 2% (w/V).

3. The biological sample extraction buffer according to claim 2, wherein the concentration of the salt ion is 0.9%-5% (w/V).

4. The biological sample extraction buffer according to claim 1, wherein the salt ion is selected from NaCl or KCl.

5. The biological sample extraction buffer according to claim 1, wherein the pH of the extraction buffer is 8.0-9.0.

6. An application of the biological sample extraction buffer according to any of claims 1 to 5 in extraction of a protein sample of a virus.

7. The purification method according to claim 6, wherein the virus is selected from SARS-CoV-2.

8. A method of use of the biological sample extraction buffer according to any of claims 1 to 5, wherein a swab sample or a liquid sample is directly added into a certain volume of a virus extraction buffer, incubation and extraction, and the extracted sample is dropwise added to a test device to interpret the result.

9. The method of use of the biological sample extraction buffer according to claim 8, wherein the swab sample or the liquid sample is incubated in the virus extraction buffer for 30s to 1min.

10. The method of use according to claim 8, wherein the test device comprises an upper cover and a lower plate, and a test strip is assembled between the upper cover and the lower plate.
